(19) Europäisches Patentamt
European Patent Office
Office européen des brevets

(11) Veröffentlichungsnummer: **0 065 197**
**A1**

# (12) EUROPÄISCHE PATENTANMELDUNG

(21) Anmeldenummer: **82103842.9**

(22) Anmeldetag: **05.05.82**

(51) Int. Cl.³: **C 07 D 319/06**, C 07 D 407/04, C 07 D 409/04, A 01 N 43/32

(30) Priorität: **14.05.81 DE 3119201**

(43) Veröffentlichungstag der Anmeldung: **24.11.82**
**Patentblatt 82/47**

(84) Benannte Vertragsstaaten: **AT BE CH DE FR GB IT LI LU NL SE**

(71) Anmelder: **BASF Aktiengesellschaft,**
**Carl-Bosch-Strasse 38, D-6700 Ludwigshafen (DE)**

(72) Erfinder: **Feuerherd, Karl-Heinz, Dr., c/o BASF Japan Ltd. C.P.O. Box 1757, Tokyo 100-91 (JP)**
Erfinder: **Rentzea, Costin, Dr., Neuenheimer Landstrasse 72, D-6900 Heidelberg (DE)**
Erfinder: **Goetz, Norbert, Dr., Schoefferstrasse 25, D-6520 Worms (DE)**
Erfinder: **Wuerzer, Bruno, Dr. Dipl.-Landwirt, Ruedigerstrasse 13, D-6701 Otterstadt (DE)**

## (54) Substituierte 1,3-Dioxane, Verfahren zu ihrer Herstellung, diese enthaltende Herbizide und ihre Anwendung als Herbizide.

(57) Die Erfindung betrifft substituierte 1,3-Dioxane der Formel

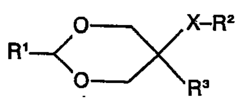
,

worin $R^1$, $R^2$, $R^3$ und X die in der Beschreibung angegebene Bedeutung besitzen, deren Herstellung und Verwendung als Herbizide.

EP 0 065 197 A1

Substituierte 1,3-Dioxane, Verfahren zu ihrer Herstellung, diese enthaltende Herbizide und ihre Anwendung als Herbizide

Die vorliegende Erfindung betrifft neue substituierte 1,3-Dioxane, Verfahren zu ihrer Herstellung, Herbizide, welche diese Verbindungen als Wirkstoffe enthalten, sowie Verfahren zur Bekämpfung unerwünschten Pflanzenwuchses durch Behandlung mit diesen Verbindungen.

Aus Tetrahedron 26 693 (1970) sind bereits 2-Phenyl-5-acetyl-5-methyl-1,3-dioxan und 2-Phenyl-5-(1'-hydroxyethyl)-5-methyl-1,3-dioxan bekannt. Dort ist aber nichts über irgendwelche Wirkungen dieser Substanzen ausgesagt.

Es wurde nun gefunden, daß substituierte 1,3-Dioxane der Formel I

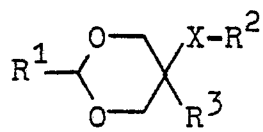

I,

in welcher

$R^1$ Furanyl, Nitrofuranyl, Thienyl, Biphenylyl, Naphthyl oder einen durch Halogen, Nitro, Cyan, Acetylamino oder gegebenenfalls durch Halogen substituiertes $C_{1-5}$-Alkyl, $C_{1-5}$-Alkoxy, $C_{1-5}$-Alkenyl, $C_{1-5}$-Alkenyloxy oder $C_{1-5}$-Dialkylamino substituierten Phenyl-, Benzyl-, Phenylalkyl-, Phenoxyalkyl- oder Cyclohexylrest oder einen durch Alkyl oder Alkoxy substituierten Cyclohexylrest oder einen Norbornenylrest bedeutet,

$R^2$ und $R^3$ gleich oder verschieden sind und einen gegebenenfalls durch Halogen oder Alkoxy substituierten Alkylrest mit 1 bis 3 C-Atomen bedeuten und

WK/P

X eine -CO-, $-\overset{N-OH}{\overset{\|}{C}}-$ , $-\overset{N-OR^4}{\overset{\|}{C}}-$ , $-\overset{OH}{\overset{|}{C}}H-$ oder $-\overset{OR^4}{\overset{|}{C}}H-$Gruppe bedeutet, in welcher $R^4$ einen gegebenenfalls durch Halogen substituierten Alkylrest mit 1 bis 4 C-Atomen oder einen $-CO-R^5$-Rest bedeutet, wobei $R^5$ einen gegebenenfalls durch Halogen, Alkoxy, Oxo (=O) oder Alkoxycarbonyl substituierten Alkylrest mit 1 bis 4 C-Atomen oder einen aromatischen Rest bedeutet,

eine selektive herbizide Wirkung haben.

Die neuen Verbindungen erhält man, indem man

A. ein 1,3-Diol der Formel II

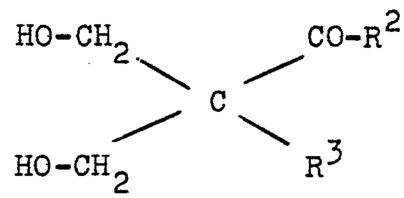

II,

worin $R^2$ und $R^3$ die oben angegebenen Bedeutungen haben,

a) mit einem Aldehyd der Formel III

$$R^1-CHO \qquad III,$$

in der $R^1$ die oben angegebene Bedeutung hat, oder

b) mit einem Acetal der Formel IV

$$R^1\text{-}CH(OR^2)_2 \qquad IV,$$

in der $R^1$ und $R^2$ die oben angegebenen Bedeutungen haben, oder

c) mit einem Aldehyd-diacetat der Formel V

$$R^1\text{-}CH(OCOCH_3)_2 \qquad V,$$

in der $R^1$ die oben angegebene Bedeutung hat, oder

d) mit einem Dihalogen-Derivat der Formel VI

$$R^1\text{-}CHY_2 \qquad VI,$$

worin $R^1$ die oben angegebene Bedeutung hat und Y Chlor oder Brom bedeutet, umsetzt oder

B. ein Keton der Formel VII

$$R^2\text{-}CH_2\text{-}CO\text{-}R^3 \qquad VII,$$

in der $R^2$ und $R^3$ die oben angegebenen Bedeutungen haben,

a) mit einer 10 bis 42 %igen wäßrigen Formaldehydlösung, oder
b) mit Paraformaldehyd oder
c) mit Dimethoxymethan, oder
d) mit 1,3,5-Trioxan und

mit einem Aldehyd der Formel II oder mit einem Aldehydacetal der Formel IV oder mit einem Aldehyddiacetat der Formel V oder mit einem Dihalogen-Derivat der Formel VI kondensiert und die so erhaltenen Ketone gegebenenfalls zum Oxim oximiert oder gegebenenfalls zum Alkohol reduziert und - falls gewünscht - danach verethert oder verestert.

Bei der Reaktion A werden die Reaktionspartner gegebenenfalls in Gegenwart eines Lösungs- oder Verdünnungsmittels und gegebenenfalls unter Zusatz einer anorganischen oder organischen Säure bei Temperaturen zwischen 20 und 150°C, gegebenenfalls unter azeotropischer Auskreisung des Reaktionswassers kondensiert. Zu den bevorzugten Lösungs- und Verdünnungsmitteln gehören Wasser, Alkohole wie Methanol, Ethanol, Isopropanol und Butanol, Kohlenwasserstoffe wie n-Hexan, n-Heptan, n- und iso-Octan, Cyclohexan, Tetrahydronaphthalin, Decahydronaphthalin, Toluol, Xylol, Cumol, halogenierte Kohlenwasserstoffe wie Methylenchlorid, Chloroform, Tetrachlorkohlenstoff, 1,2-Dichlorethan und Chlorbenzol; Ether wie Diethylether, Dibutylether, Tetrahydrofuran und 1,4-Dioxan; Amide wie Dimethylformamid und Dimethylacetamid oder entsprechende Gemische.

Geeignete anorganische und organische Säuren sind beispielsweise Salzsäure, Bromwasserstoffsäure, Schwefelsäure, Benzolsulfonsäure, p-Toluolsulfonsäure, Dodecylbenzolsulfonsäure und Trifluormethylsulfonsäure.

Die Umsetzung wird im allgemeinen bei Temperaturen zwischen 20 und 150°C, drucklos oder unter Druck kontinuierlich oder diskontinuierlich durchgeführt.

1,3-Diole der Formel II sind bekannt und beispielsweise von G. Morgan und C.F. Griffith in J.Chem.Soc. 841 (1937) beschrieben. Sie lassen sich nach den dort beschriebenen Verfahren bzw. nach entsprechend abgewandelten Methoden herstellen.

Die Reaktion B wird gegebenenfalls in Gegenwart eines Lösungs- oder Verdünnungsmittels und gegebenenfalls unter Zusatz einer anorganischen oder organischen Base und/oder einer anorganischen oder organischen Säure bei Temperaturen zwischen 20 und 150°C, gegebenenfalls unter azeotropischer Auskreisung des Reaktionswassers durchgeführt.

Als Lösungs- und Verdünnungsmittel sind Wasser, Alkohole wie Methanol, Ethanol, Isopropanol und Butanol, Kohlenwasserstoffe wie n-Hexan, n-Heptan, n- und iso-Octan, Cyclohexan, Petrolether, Benzol, Toluol, Xylol, Cumol; halogenierte Kohlenwasserstoffe wie Methylenchlorid, Chloroform, Tetrachlorkohlenstoff, 1,2-Dichlorethan und Chlorbenzol; Ether wie Diethylether, Dibutylether, Tetrahydrofuran, 1,n-Dioxan, 1,2-Dimethoxyethan und Anisol; Amide wie Dimethylformamid und Dimethylacetamid oder entsprechende Gemische bevorzugt.

Geeignete Basen und Säuren, die gegebenenfalls in katalytischen bis zu stöchiometrischen Mengen verwendet werden, sind beispielsweise Alkali- und Erdalkalihydroxide, wie Natrium-, Kalium-, Calcium- und Bariumhydroxid, Alkoxide wie Natrium- und Kaliummethoxid, -ethoxid, -isopropoxid und tert.-butoxid, Amine wie Triethylamin, N,N-Dimethylcyclohexylamin oder Pyridin, ferner anorganische Säuren wie Salzsäure, Phosphorsäure und Schwefelsäure oder organische Säuren wie Ameisensäure, p-Toluolsulfonsäure oder Trifluormethylsulfonsäure.

Die Umsetzungen werden im allgemeinen bei Temperaturen zwischen 20 und 150°C, drucklos oder unter Druck, kontinuierlich oder diskontinuierlich durchgeführt.

Bevorzugte herbizide Verbindungen der allgemeinen Formel I sind diejenigen, worin die Gruppe $R^1$ 2-Furanyl, 5-Nitrofuran-2-yl, 2- und 3-Thienyl, 1- und 2-Naphthyl, Phenyl, 2-, 3- und 4-Fluor, Chlor- und Bromphenyl, 2- und 4-Methylphenyl, 2-, 3- und 4-Methoxyphenyl, 4-Tetrafluorethoxyphenyl, 3- und 4-Trifluormethylphenyl, 2- und 4--Nitrophenyl, 4-tert-Butylphenyl, 4-Acetylaminophenyl, 4-Dimethylaminophenyl, 2,4-Dichlorphenyl, 3,4-Dichlorphenyl, 2-Chlor-6-nitrophenyl, 4-Methyl-3-nitrophenyl, ferner Benzyl, 1-Phenyl-1-ethyl, Phenoxymethyl, Cyclohexyl, Cyclohexenyl, 4-Methoxy-cyclohexyl und Norbornenyl bedeutet, $R^2$ und $R^3$ Methyl, Chlormethyl, Brommethyl, Methoxymethyl, Ethyl, 1-Chlorethyl und 1-Bromethyl bedeuten und X eine -CO-, -CH(OH)-, -C=NOH-, $-CH(OR^4)-$ oder $-C=NOR^4-$Gruppe bedeutet, wobei $R^4$ bevorzugt für Methyl, Ethyl, n-Propyl, n-Butyl, 4-Chlorbutyl, Allyl, 2-Buten-1-yl, Acetyl, Chloracetyl, Propionyl, Butyryl, Isobutyryl, Methoxyacetyl und Benzoyl steht.

Bevorzugte herbizide Verbindungen der allgemeinen Formel I sind weiterhin diejenigen, worin die Gruppen $R^1$ und $-X-R^2$ eine cis-Konfiguration einnehmen. Die oben angegebene cis-Konfiguration tritt dann auf, wenn die Gruppe $R^1$ in äquatorialer Stellung und die Gruppe $-X-R^2$ in axialer Stellung stehen.

Bei der Verwendung der erfindungsgemäßen Verbindungen als Herbizide kann man die aktiveren cis-Isomeren zusammen mit den trans-Isomeren verwenden und die Mischungen können sogar einen überwiegenden Anteil der letzteren Isomeren enthalten. Je höher der Gehalt an cis-Isomeren ist, um so

höher ist die herbizide Wirkung einer gegebenen Mischung aus cis- und trans-Isomeren. Besonders günstig ist es, wenn die cis-Verbindung in einer Menge vorhanden ist, die mindestens gleich der der entsprechenden trans-Verbindung ist. Vorzugsweise liegt das cis:trans-Verhältnis höher als 1:1, insbesondere höher als 3:2 und ganz bevorzugt höher als 3:1.

Die folgenden Versuche erläutern die Herstellung der Wirkstoffe.

Beispiel 1

Eine Lösung von 108 g (1,49 Mol) Methylethylketon und 1,5 g Lithiumhydroxid in 600 ml Wasser wurde mit 300 g einer 30 %igen wäßrigen Formaldehydlösung tropfenweise versetzt. Es wurde 12 Stunden nachgerührt. Nach Zugabe von 20 g p-Toluolsulfonsäurehydrat, 1000 ml Toluol und 162 g (1,49 Mol) 3-Thiophenaldehyd wurde das Gemisch auf 115°C erwärmt und das Wasser azeotropisch ausgekreist. Nach Abkühlen auf 20°C wurden nacheinander 100 ml 35 %ige Natriumhydrogensulfitlösung und 500 ml Wasser zugegeben, 30 Minuten nachgerührt und die organische Phase abgetrennt, getrocknet und eingeengt. Der feste Rückstand wurde mit 50 ml Ethanol dispergiert und die Kristalle wurden abgesaugt. Man erhielt 70,7 g (21 % d.Th.) 2-(3'-Thienyl)-5-acetyl-5-methyl-1,3-dioxan als weiße Kristalle vom Schmelzpunkt 93°C.

Beispiel 2

Die Lösung von 21 g (0,093 Mol) 2-(3'-Thienyl)-5-acetyl-5-methyl-1,3-dioxan in 200 ml Methanol wurde bei 20°C portionsweise mit 4,9 g (0,13 Mol) $NaBH_4$ versetzt und 2 Stunden bei 60°C nachgerührt. Nach Abkühlen auf 20°C

wurde das Gemisch mit der Lösung von 1 g Natriumhydroxid in 30 ml Wasser versetzt und eingeengt. Der Rückstand wurde in 300 ml Methylenchlorid gelöst, nacheinander mit 1 $NH_2SO_4$ und mit Wasser gewaschen, über $Na_2SO_4$ getrocknet und eingeengt.

Man erhielt 20 g (92,5 % d.Th.) 2-(3'-Thienyl)-5-(1'-hydroxyethyl)-5-methyl-1,3-dioxan vom Schmelzpunkt 77-79°C.

Beispiel 3

Einer Lösung von 42 g (0,58 Mol) Hydroxylaminhydrochlorid und 84 g (0,62 Mol) Natriumacetattrihydrat in 210 ml Wasser wurde die Lösung von 75,5 g (0,34 Mol) 2-Phenyl-5--acetyl-5-methyl-1,3-dioxan (Fp = 103°C; hergestellt nach T.A. Crabb und R.F. Newton, Tetrahedron 26 (1970)) in 150 ml Dioxan bei 25°C zügig zugetropft. Nach zweistündigem Rückfluß wurde das Gemisch abgekühlt und mit je 150 ml Methylenchlorid dreimal extrahiert. Die organische Phase wurde mit Wasser gewaschen, getrocknet und eingeengt. Der Rückstand wurde mit 100 ml Petrolether digeriert und abgesaugt. Man erhielt 64 g (80 %) 2-Phenyl-5-(1'-hydroxyiminoethyl)-5-methyl-1,3-dioxan als farblose Kristalle vom Schmelzpunkt 165 bis 167°C.

Beispiel 4

Die Lösung von 16,5 g (0,07 Mol) 2-Phenyl-5-(1'-hydroxyiminoethyl)-5-methyl-1,3-dioxan und 3 Tropfen Dibutylzinndilaurat in 150 ml trockenem Aceton wurden mit 5,4 g (0,095 Mol) Methylisocyanat tropfenweise versetzt. Nach zweitägigem Rühren bei 25°C wurde das Gemisch in 500 ml Eiswasser eingerührt, mit 2 N HCl auf pH 2 eingestellt und 30 Minuten gerührt. Nach dreimaliger Extraktion mit je 100 ml Methylenchlorid wurden die vereinigten Extrak-

te über $MgSO_4$ getrocknet und eingeengt. Man erhielt 14,4 g (70,5 %) 2-Phenyl-5-[1'-(methylcarbamoyloxyimino)-ethyl]-5-methyl-1,3-dioxan vom Schmelzpunkt 112-114°C.

Beispiel 5

Die Lösung von 16,7 g (0,075 Mol) 2-Phenyl-5-methyl-5-(1'-hydroxyethyl)-1,3-dioxan (Fp = 67°C, hergestellt nach T.A. Crabb und R.F. Newton, loc.cit.) und 0,5 g 4-Dimethylaminopyridin in 70 g Propionanhydrid wurden bei 70°C 16 Stunden gerührt und anschließend im Vakuum eingeengt. Der Rückstand wurde in 300 ml Ether gelöst und mit 300 ml 7 %iger $NaHCO_3$-Lösung 3 Stunden gerührt. Die organische Phase wurde abgetrennt, dreimal mit Wasser gewaschen, eingeengt und im Vakuum, schließlich bei 80°C und 1 mbar getrocknet. Man erhielt 15,9 g (76,3 %) 2-Phenyl-5-methyl-5-(1'-propionyloxyethyl)-1,3-dioxan als farbloses Harz.

Beispiel 6

Die Lösung von 16,7 g (0,075 Mol) 2-Phenyl-5-methyl-5-(1'-hydroxyethyl)-1,3-dioxan (Fp = 67°C) und 5,5 g Tetrabutylammoniumhydrogensulfat in 130 ml n-Pentylchlorid wurden bei 50°C mit 89 g 50 %iger Natronlauge 5 Tage gerührt, anschließend mit 1 l Wasser vermischt und mit je 70 ml Methylenchlorid fünfmal extrahiert. Die organischen Extrakte wurden dreimal mit je 80 ml Wasser gewaschen, über $Na_2SO_4$ getrocknet und im Vakuum destilliert. Man erhielt 15,2 g (69 %) 2-Phenyl-5-methyl-5-(1'-pentyloxyethyl)-1,3-dioxan als farblose Flüssigkeit vom Siedepunkt 145-150°C (0,4 mbar) und $n_D^{28}$ = 1,5012.

In entsprechender Weise konnten die in der folgenden Tabelle aufgeführten Verbindungen hergestellt werden:

Tabelle

| Beispiel Nummer | $R^1$ | X | $R^2$ | $R^3$ | Fp °C | IR [Film] ($cm^{-1}$) |
|---|---|---|---|---|---|---|
| 7 | $2-Cl-C_6H_4-$ | -CO- | $CH_3-$ | $CH_3-$ | 99-104 | |
| 8 | $4-Cl-C_6H_4-$ | -CO- | $CH_3-$ | $CH_3-$ | 142-144 | |
| 9 | $2,4-DiCl-C_6H_3-$ | -CO- | $CH_3-$ | $CH_3-$ | 75- 81 | |
| 10 | $C_6H_5-$ | -CO- | $CH_3-$ | $C_2H_5-$ | Harz | |
| 11 | $2,6-DiCl-C_6H_3-$ | -CO- | $CH_3-$ | $CH_3-$ | 125-130 | |
| 12 | 2-Furanyl | -CO- | $CH_3-$ | $CH_3-$ | 85- 88 | |
| 13 | $4-CH_3O-C_6H_4-$ | -CO- | $CH_3-$ | $CH_3-$ | 166-168 | |
| 14 | $4-CH_3-C_6H_4-$ | -CO- | $CH_3-$ | $CH_3-$ | 123-125 | |
| 15 | 4-tert.but-$C_6H_4-$ | -CO- | $CH_3-$ | $CH_3-$ | 111-113 | |
| 16 | $C_6H_5-CH(CH_3)-$ | -CO- | $CH_3-$ | $CH_3-$ | Harz | 3118,2960,2840,1700, 1448,1382,1152,1084, 1030,926,960. |
| 17 | $4-NO_2-C_6H_4-$ | -CO- | $CH_3-$ | $CH_3-$ | 50- 56 | |
| 18 | $2-NO_2-C_6H_4-$ | -CO- | $CH_3-$ | $CH_3-$ | Harz | 2960,1700,1525,1382, 1352,1205,1110,1092, 1073,1010,845. |
| 19 | $2-CH_3-C_6H_4-$ | -CO- | $CH_3-$ | $CH_3-$ | 80- 83 | |
| 20 | $C_6H_5-CH-CH_2$<br>$CH_3$ | -CO- | $CH_3-$ | $CH_3-$ | Harz | 2950,2850,1700,1448, 1352,1140,1090,1068, 1020,760,700. |
| 21 | $CH_3$<br>$C_6H_5-O-CH-$ | -CO- | $CH_3-$ | $CH_3-$ | Harz | 2965,2845,1700,1590, 1240,1165,1110,1095, 752,690. |
| 22 | $C_6H_5-CH_2-$ | -CO- | $CH_3-$ | $CH_3-$ | 80- 84 | |
| 23 | $4-(CH_3CO-NH)-C_6H_4-$ | -CO- | $CH_3-$ | $CH_3-$ | 123-124 | |

<u>Tabelle:</u> Fortsetzung

| Beispiel Nummer | $R^1$ | X | $R^2$ | $R^3$ | Fp °C | IR [Film] ($cm^{-1}$) |
|---|---|---|---|---|---|---|
| 24 | $4-[(CH_3)_2N]-C_6H_4-$ | -CO- | $CH_3-$ | $CH_3-$ | 104-106 | |
| 25 | Cyclohexyl | -CO- | $CH_3-$ | $CH_3-$ | Harz | 2915,2840,1700,1446, 1386,1352,1152,1125, 1084,1030,886. |
| 26 | $CH_3$<br>$4-(tert.but)-C_6H_4-CH_2-CH-$ | -CO- | $CH_3-$ | $CH_3-$ | Harz | 2950,2854,1702,1505, 1455,1386,1360,1265, 1150,1086,920. |
| 27 | $4-(HCF_2-CF_2-O)-C_6H_4-$ | -CO- | $CH_3-$ | $CH_3-$ | Harz | 2960,2845,1702,1504, 1382,1350,1300,1270, 1190,1115,1090. |
| 28 | 5-Nitro-2-furanyl | -CO- | $CH_3-$ | $CH_3-$ | 60 | |
| 29 | $3-NO_2$, $4-CH_3-C_6H_3-$ | -CO- | $CH_3-$ | $CH_3-$ | 64- 65 | |
| 30 | $2-Cl-6-NO_2-C_6H_3-$ | -CO- | $CH_3-$ | $CH_3-$ | 83- 87 | |
| 31 | $C_6H_5-$ | $O-CO-CH_3$<br>-CH- | $CH_3-$ | $CH_3-$ | Harz | |
| 32 | $C_6H_5-$ | $O(CH_2)_2-CH_3$<br>-CH- | $CH_3-$ | $CH_3-$ | 81 | $n_D^{20}$ = 1,4938 |
| 33 | $C_6H_5-$ | $O(CH_2)_3-CH_3$<br>-CH- | $CH_3-$ | $CH_3-$ | 81 | $n_D^{28}$ = 1,4958 |
| 34 | $C_6H_5-$ | $O-(CH_2)_4-Cl$<br>-CH- | $CH_3-$ | $CH_3-$ | 81 | $n_D^{27}$ = 1,5080 |
| 35 | $C_6H_5-$ | $O-CO-NH-CH_3$<br>-CH- | $CH_3-$ | $CH_3-$ | 68- 70 | |

<u>Tabelle</u>: Fortsetzung

| Beispiel Nummer | $R^1$ | X | $R^2$ | $R^3$ | Fp °C | IR [Film] ($cm^{-1}$) |
|---|---|---|---|---|---|---|
| 36 | $C_6H_5$- | O-CO-NH-$C_3H_7$-i<br>\|<br>-CH- | $CH_3$- | $CH_3$- | 124-126 | |
| 37 | 2,4-DiCl-$C_6H_3$ | N-OH<br>‖<br>-C- | $CH_3$- | $CH_3$- | 148-153 | |
| 38 | $C_6H_5$- | N-OH<br>‖<br>-C- | $CH_3$- | $CH_3$- | 165-167 | |
| 39 | $C_6H_5$- | N-O-$COCH_3$<br>‖<br>-C- | $CH_3$- | $CH_3$- | Harz | 2960,1760,1385,1360, 1240,1205,1115,1092, 1004,926,750. |
| 40 | $C_6H_5$- | N-O-CO-$C_2H_5$<br>‖<br>-C- | $CH_3$- | $CH_3$- | Harz | 2965,1754,1450,1386, 1165,1136,1116,1092, 1070,1010,868. |
| 41 | $C_6H_5$- | N-O-CO-NH-$C_3H_7$-n<br>‖<br>-C- | $CH_3$- | $CH_3$- | 101-104 | |
| 42 | $C_6H_5$- | N-O-CO-NH-$C_3H_7$-i<br>‖<br>-C- | $CH_3$- | $CH_3$- | 83 | |
| 43 | $C_6H_5$- | N-O-CO-NH-$CH_3$<br>‖<br>-C- | $CH_3$- | $CH_3$- | 112-114 | |
| 44 | 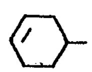 | -CO- | $CH_3$- | $CH_3$- | Harz | 3120,2960,2905,2840, 1700,1354,1154,1085, 1035. |
| 45 | 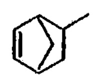 | -CO- | $CH_3$- | $CH_3$- | Harz | 3050,2960,2855,1701, 1385,1350,1332,1150, 1035,1006,720. |
| 46 | 3-Br-4-$CH_3O$-$C_6H_3$- | -CO- | $CH_3$- | $CH_3$- | Harz | 2960,2840,1700,1602, 1498,1455,1382,1260, 1088,1050,815. |
| 47 | $C_6H_5$- | -CO- | -$CH_2Br$ | $CH_3$- | Harz | |

Die Anwendung der Wirkstoffe erfolgt z.B. in Form von direkt versprühbaren Lösungen, Pulvern, Suspensionen - auch hochprozentige wässerige, ölige oder sonstige Suspensionen -oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken.

Zur Herstellung von direkt versprühbaren Lösungen, Emulsionen, Pasten und Öldispersionen kommen Mineralölfraktionen von mittlerem mit hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformid, Dimethylsulfoxid, N-Methylpyrrolidon, Wasser usw. in Betracht.

Wässerige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier-oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen: Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol-, Octylphenol-, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz und Nußschalenmehl, Cellulosepulver und andere feste Trägerstoffe.

Die Formulierungen enthalten zwischen 0,1 und 95 Gewichtsprozent Wirkstoff, vorzugsweise zwischen 0,5 und 90 Gewichtsprozent.

Die Wirkstoffe werden angewendet, beispielsweise durch Gießen, Streuen, Stäuben, Spritzen oder Sprühen auf die Pflanzen oder den Boden, durch Injizieren oder Bestreichen von Pflanzen oder durch Einbringen in das Bewässerungswasser.

Beispiel I

Man vermischt 90 Gewichtsteile der Verbindung des Beispiels 1 mit 10 Gewichtsteilen N-Methyl-alpha-pyrrolidon und erhält eine Lösung, die zur Anwendung in Form kleinster Tropfen geeignet ist.

Beispiel II

10 Gewichtsteile der Verbindung des Beispiels 5 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-mono-ethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

Beispiel III

20 Gewichtsteile der Verbindung des Beispiels 12 werden in einer Mischung gelöst, die aus 40 Gewichtsteilen Cyclohexanon, 30 Gewichtsteilen Isobutanol, 20 Gewichtsteilen

des Anlagerungsproduktes von 7 Mol Ethylenoxid an 1 Mol Isooctylphenol und 10 Gewichteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel IV

20 Gewichtsteile der Verbindung des Beispiels 31 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel V

20 Gewichtsteile des Wirkstoffs gemäß Beispiel 2 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel VI

95 Gewichtsteile der Verbindung des Beispiels 31 werden mit 5 Gewichtsteilen feinteiligem Kaolin innig vermischt. Man erhält auf diese Weise ein Stäubemittel, das 95 Gewichtsprozent des Wirkstoffs enthält.

Beispiel VII

30 Gewichtsprozent der Verbindung des Beispiels 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel VIII

40 Gewichtsteile des Wirkstoffs des Beispiels 12 werden mit 10 Teilen Natriumsalz eines Phenolsulfonsäure-harnstoff-formaldehyd-kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

Beispiel IX

20 Teile des Wirkstoffs des Beispiels 10 werden mit 12 Teilen Calciumsalz der Dodecylbenzolsulfonsäure, 8 Teile Fettalkohol-polyglykolether, 2 Teilen Natriumsalz eines Phenolsulfonsäure-harnstoff-formaldehyd-kondensats und 68 Teilen eines paraffinischen Mineralöls innig vermischt. Man erhält eine stabile ölige Dispersion.

Die Applikation kann im Vorauflaufverfahren oder bei Nachauflaufanwendung erfolgen. Vorzugsweise werden die neuen Wirkstoffe nach dem Auflaufen der unerwünschten Pflanzen, sowohl auf Kulturflächen als auch auf unbebautem Land, ausgebracht. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit und Wachstumsstadium 0,1 bis 15 kg/ha und mehr.

Der Einfluß der neuen substituierten 1,3-Dioxane auf das Wachstum von Pflanzen wird anhand von Gewächshausversuchen vorgeführt:

Als Kulturgefäße dienten Plastikblumentöpfe mit 300 $cm^3$ Inhalt und lehmiger Sand mit etwa 1,5 % Humus als Substrat. Die Samen der Testpflanzen wurden nach Arten getrennt flach eingesät. Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düsen gespritzt. Bei dieser Applikationsmethode handelt es sich um eine Aufwandmenge entsprechend 3,0 kg Wirkstoff/ha. Nach dem Aufbringen der Mittel wurden die Gefäße leicht beregnet, um Keimung und Wachstum in Gang zu bringen. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern dies nicht durch die Wirkstoffe beeinträchtigt wurde.

Die Aufstellung der Versuche erfolgte im Gewächshaus, wobei für wärmeliebende Arten wärmere Bereiche (20 bis 40°C) und für solche gemäßigter Klimate 10 bis 20°C bevorzugt wurden. Die Versuchsperiode erstreckte sich über 2 bis 4 Wochen. Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung und normaler Ablauf und 100 kein Aufgang der Pflanzen bzw. völlige Zerstörung zumindest der oberirdischen Teile.

Die Gewächshausversuche ergeben, daß z.B. die Verbindungen der Beispiele Nr. 5, 10, 39 und 40 bei Vorauflaufanwendung von 3,0 kg Wirkstoff/ha eine herbizide Wirkung besitzen.

Neben der bevorzugten Vorauflaufanwendung ist auch eine Nachauflaufbehandlung (Blattbehandlung) möglich.

In Anbetracht der guten Verträglichkeit der Wirkstoffe und der Vielseitigkeit der Applikationsmethoden können die neuen Verbindungen oder diese enthaltende Mittel noch in einer weiteren großen Zahl von Kulturpflanzen zur Beseitigung unerwünschten Pflanzenwuchses eingesetzt werden.

Zur Verbreiterung des Wirkungsspektrums und zur Erzielung synergistischer Effekte können die neuen 1,3-Dioxane sowohl unter sich als auch mit zahlreichen Vertretern anderer herbizider oder wachstumsregulierender Wirkstoffgruppen gemischt und gemeinsam ausgebracht werden.

Beispielsweise kommen als Mischungspartner Diazine, 4 H-3, 1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Zur Aktivierung der herbiziden Wirkung können auch Netz- und Haftmittel sowie nicht phytotoxische Öle und Ölkonzentrate zugesetzt werden.

Patentansprüche

1. Substituierte 1,3-Dioxane der Formel I

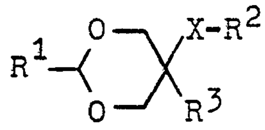

I,

in welcher

$R^1$ Furanyl, Nitrofuranyl, Thienyl, Biphenylyl, Naphthyl oder ein gegebenenfalls durch Halogen, Nitro, Cyan, Acetylamino oder gegebenenfalls durch Halogen substituiertes $C_{1-5}$-Alkyl, $C_{1-5}$-Alkoxy, $C_{1-5}$-Alkenyl, $C_{1-5}$-Alkenyloxy oder $C_{1-5}$-Dialkylamino substituierten Phenyl-, Benzyl-, Phenylalkyl-, Phenoxyalkyl- oder Cyclohexylrest oder einen durch Alkyl oder Alkoxy substituierten Cyclohexylrest oder einen Cyclohexenylrest oder einen Norbornenylrest bedeutet,

$R^2$ und $R^3$ gleich oder verschieden sind und einen gegebenenfalls durch Halogen oder Alkoxy substituierten Alkylrest mit 1 bis 3 C-Atomen bedeuten und

X eine -CO-, $-C(=N-OH)-$, $-C(=N-OR^4)-$, $-CH(OH)-$ oder $-CH(OR^4)-$-Gruppe bedeutet, in welcher $R^4$ einen gegebenenfalls durch Halogen substituierten Alkylrest mit 1 bis 4 C-Atomen oder einen $-CO-R^5$-Rest bedeutet, wobei $R^5$ einen gegebenenfalls durch Halogen, Alkoxy, Oxo (=O) oder Alkoxycarbonyl substituierten Alkylrest mit 1 bis 4 C-Atomen oder einen aromatischen Rest bedeutet.

2. Verbindungen der Formel I gemäß Anspruch 1, worin die Gruppe $R^1$ 2-Furanyl, 5-Nitrofuran-2-yl, 2- und 3--Thienyl, 1- und 2-Naphthyl, Phenyl, 2-, 3- und 4-Fluor, Chlor- und Bromphenyl, 2- und 4-Methylphenyl, 2-, 3- und 4-Methoxyphenyl, 4-Tetrafluorethoxyphenyl, 3- und 4-Trifluormethylphenyl, 2- und 4-Nitrophenyl, 4-tert-Butylphenyl, 4-Acetylaminophenyl, 4-Dimethylaminophenyl, 2,4-Dichlorphenyl, 3,4-Dichlorphenyl, 2-Chlor-6-nitrophenyl, 4-Methyl-3-nitrophenyl, ferner Benzyl, 1-Phenyl-1-ethyl, Phenoxymethyl, Cyclohexyl, Cyclohexenyl, 4-Methoxy-cyclohexyl und Norbornenyl bedeutet, $R^2$ und $R^3$ Methyl, Chlormethyl, Brommethyl, Methoxymethyl, Ethyl, 1-Chlorethyl und 1--Bromethyl bedeuten und X eine -CO-, -CH(OH)-, -C=NOH-, $-CH(OR^4)-$ oder $-C{=}NOR^4$-Gruppe bedeutet, wobei $R^4$ bevorzugt für Methyl, Ethyl, n-Propyl, n-Butyl, 4-Chlorbutyl, Allyl, 2-Buten-1-yl, Acetyl, Chloracetyl, Propionyl, Butyryl, Isobutyryl, Methoxyacetyl und Benzoyl steht.

3. Verfahren zur Herstellung von Verbindungen der Formel I gemäß Anspruch 1, dadurch gekennzeichnet, daß man

A. ein 1,3-Diol der Formel II

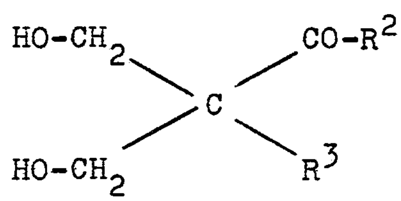

II,

worin $R^2$ und $R^3$ die oben angegebenen Bedeutungen haben,

a) mit einem Aldehyd der Formel III

$$R^1\text{-CHO} \qquad III,$$

in der $R^1$ die oben angegebene Bedeutung hat, oder

b) mit einem Acetal der Formel IV

$$R^1\text{-CH}(OR^2)_2 \qquad IV,$$

in der $R^1$ und $R^2$ die oben angegebenen Bedeutungen haben, oder

c) mit einem Aldehyd-diacetat der Formel V

$$R^1\text{-CH}(OCOCH_3)_2 \qquad V,$$

in der $R^1$ die oben angegebene Bedeutung hat, oder

d) mit einem Dihalogen-Derivat der Formel VI

$$R^1\text{-CHY}_2 \qquad VI,$$

worin $R^1$ die oben angegebene Bedeutung hat und Y Chlor oder Brom bedeutet, umsetzt oder

B. ein Keton der Formel VII

$$R^2\text{-CH}_2\text{-CO-}R^3 \qquad VII,$$

in der $R^2$ und $R^3$ die oben angegebenen Bedeutungen haben,

a) mit einer 10 bis 42 %igen wäßrigen Formaldehydlösung, oder

b) mit Paraformaldehyd oder

c) mit Dimethoxymethan, oder

d) mit 1,3,5-Trioxan und

mit einem Aldehyd der Formel II oder mit einem Aldehydacetal der Formel IV oder mit einem Aldehyddiacetat der Formel V oder mit einem Dihalogen-Derivat der Formel VI kondensiert und die so erhaltenen Ketone gegebenenfalls zum Oxim oximiert oder gegebenenfalls zum Alkohol reduziert und - falls gewünscht - danach verethert oder verestert.

4. Herbizid, enthaltend mindestens eine Verbindung gemäß Anspruch 1.

5. Herbizid, enthaltend mindestens eine Verbindung gemäß Anspruch 1 und einen flüssigen oder festen Trägerstoff.

6. Verwendung einer Verbindung gemäß Anspruch 1 zur Bekämpfung unerwünschten Pflanzenwuchses.

7. Verfahren zur Bekämpfung unerwünschten Pflanzenwuchses, dadurch gekennzeichnet, daß man die Pflanzen oder den Boden mit einer Verbindung gemäß Anspruch 1 behandelt.

Europäisches Patentamt

**EUROPÄISCHER RECHERCHENBERICHT** 0065197 Nummer der Anmeldung

EP 82 10 3842

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| D,X | TETRAHEDRON, Band 26, 1970, Seiten 693-699, Oxford (GB); T.A.CRABB et al.: "The NMR spectra and configurations of some 2,5,5-trisubstituted 1,3-dioxans". *Seiten 693-694,696, Experimental* | 1-3 | C 07 D 319/06<br>C 07 D 407/04<br>C 07 D 409/04<br>A 01 N 43/32 |
| X | DE-A-2 454 281 (ALLEN & HANBURYS) *Seiten 1-5,7,8,12-14,16-20,27-30* | 1-3 | |
| A | CHEMICAL ABSTRACTS, Band 66, 1967, Seite 9736, Nr. 104268x, Columbus Ohio (USA); A.V.BOGATSKII et al.: "Antimicrobial and fungicidal activity of 2,5-dialkyl-5alpha-alkoxyethyl-1,3-dioxanes". *Zusammenfassung* | 1,4 | RECHERCHIERTE SACHGEBIETE (Int. Cl. ³) |
| A | US-A-3 792 062 (EUGENE G. TEACH) *Spalten 1-9* | 1-7 | C 07 D 319/00<br>C 07 D 407/00<br>C 07 D 409/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 21-08-1982 | FRANCOIS J.C.L. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82